(19) Europäisches Patentamt
European Patent Office
Office européen des brevets

(11) EP 1 295 950 A1

(12) # EUROPEAN PATENT APPLICATION

(43) Date of publication:
**26.03.2003 Bulletin 2003/13**

(51) Int Cl.[7]: **C12Q 1/68**

(21) Application number: **01203617.4**

(22) Date of filing: **25.09.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **GT Diagnostics B.V.**
**6708 PD Wageningen (NL)**

(72) Inventors:
- **Langeveld, Simon Albertus**
**2121 AX Bennebroek (NL)**
- **van der Kop, Diane Antoinette Maria**
**6705 DJ Wageningen (NL)**
- **de Boer, Anne Douwe**
**6621 AC Dreumel (NL)**

(74) Representative: **Prins, Adrianus Willem et al**
**Vereenigde,**
**Nieuwe Parklaan 97**
**2587 BN Den Haag (NL)**

Remarks:
The sequence listing, which is published as annex to the application documents, was filed after the date of filing. The applicant has declared that it does not include matter which goes beyond the content of the application as filed.

(54) **Expression profiling**

(57) The invention relates to DNA sequencing methods useful in expression profiling. The invention provides a semi-automated high throughput testing facility for expression profiling with immediate use in expression profiling such as in testing plant quality of horticultural and agricultural products

EP 1 295 950 A1

## Description

The invention relates to methods useful in expression profiling.

Criteria for quality and environmental safety of fresh products are getting more and more strict due to a changing awareness of consumers, trade and agricultural and horticultural industry. A parallel development is the increasing need for quality tests. A clear market exists for objective detection methods that will enable quality ranking. By using such tests it will become possible to predict quality decay, to certify product batches and to monitor the effect of external treatments. Tests are necessary to monitor quality during handling, transport and storage for food products like potatoes, vegetables and fruit, or horticultural products like trees and shrubs, pot plants and cut flowers. Tests are also necessary to predict the time of harvest or planting for food and horticultural products. In addition, tests are necessary to monitor quality in specialized situations, e.g. to monitor the time of low temperature treatment to induce flowering in various bulbous species or to monitor the exposure to air pollution in indicator species. Until recently, quality was not or only poorly defined and was usually only judged by visually examining the product, usually based on subjective criteria, that were difficult to quantify. An innovation for the development of objective tests, however, is the use of modern biotechnology. The recent advances in equipment for detection and analysis combined with novel molecular and biochemical techniques (e.g. functional genomics) to identify and isolate markers that typify a defined physiological stage definitely provide new opportunities for the development of sensitive tests.

Genomics and bio-informatics rapidly provide a growing number of markers for a large array of products. The development of equipment that is able to use this information for diagnostic purposes becomes increasingly important.

The invention provides a semi-automated high throughput testing facility for expression profiling with immediate use in testing plant quality of horticultural and agricultural products which can be executed in one reaction vessel per profile tested. However, a method according to the invention is also applicable to other organisms where expression profiling may be at order. Expression profiling is in general done by annealing a known but quantitatively to be detected marker nucleic acid with a detecting nucleic acid, which most times is provided with a label. Other systems use an array format, wherein the nucleic acid(s) to be detected adhere to an array. Here we provide a method for expression profiling wherein the nucleic acid(s) to be quantified are at least partly sequenced. Preferably, the test is based on the detection of RNA expression of a set of biomarkers. In principle, this approach is universally applicable, since biomarkers can be used for every quality determination with a physiological background. For every class of problems concerning quality however, markers must be identified and isolated. With quality loss, e.g. stress-induced senescence, oxidative damage or desiccation, the plant tissues will go through various physiological stages, in which different genes are switched on or off. The levels of expression of these marker genes reflect the physiological stage and therefore the condition and quality of the plant and/or product. In general the invention provides a method for determining a developmental or physiological stage of an organism by determining a gene expression profiles of said organism or parts (such as cells or cell extracts) thereof, said method at least comprising determining the expression of at least a first gene and a second gene, or fragments thereof, involved in development of said organism, said method comprising a) providing a first (single-stranded) nucleic acid template derived from said first gene and a second (single-stranded) nucleic acid template derived from said second gene b) hybridising at least one primer to said first template and at least one other primer to said second template c) and determining binding of said primers to said templates in one reaction vessel. With reaction vessel, an entity, such as a droplet or test tube, in which a reaction takes place is meant. In a preferred embodiment, a method is provided wherein a template comprises RNA. For fresh products, quality usually is a reflection of the physiological condition of the product. There is a direct relation between the pattern of gene expression on the RNA and protein level, and the physiological status of a cell. Therefore, it is possible to isolate marker RNA's that are indicative for the physiological changes and as a consequence for the quality of cells, tissues or organisms. RNA's are for example extracted from tissues by use of organic solvents and optionally subsequently separated from DNA in a selective precipitation step, for example including a high molar solutions of LiCl. Reverse transcription of RNA into cDNA is performed by a reverse transcriptase step such as by using avian myeblastosis virus (AMV) reverse transcriptase using oligodT- mers or gene specific oligonucleotides to prime the synthesis of cDNA in a reaction with polyA mRNA or total RNA. Amplification of part(s) of any marker gene can subsequently be performed in a polymerase chain reaction (PCR) using template specific primers to enable detection by the method described below.

It is preferred that said method includes a sequencing reaction that can be initiated by a primer, preferably, said primer can initiate a sequencing reaction carried out by a DNA-polymerase. It is also provided to use a DNA polymerase that is RNA dependent. In a preferred embodiment, the invention provides a method wherein a dATP or ddATP analogue is used which is capable of acting as a substrate for said polymerase but incapable of acting as a substrate for a pyrophosphate detection enzyme such as ATP-sulfurylase, such as is known from a method of sequencing DNA generally called pyrosequencing (WO/98/13523). Pyrosequencing is originally developed to sequence large amounts of short to medium length DNA sequences Using this technique single nucleotide polymorphism's (SNPs) can be detected in a simple way and in large quantities. SNPs have been developed for use in genetic studies and are currently being implemented in medical diagnostics. In the pyrosequecing reaction DNA polymerase catalyzes the incorporation of a deoxynucleotide triphosphate, complementary to template nucleotide base, into the new DNA strand, while releasing a pyrophosphate. Via several enzymatic steps the pyrophosphate is converted into light. The amount of light is proportional to the number of nucleotides incorporated at that position and therefore proportional to the amount of matching template. After each reaction the surplus of substrate is removed. Pyrosequencing therefore results in simultaneous determination of the sequence and quantification of the different SNPs in the template mix.
That the method of pyrosequencing is also applicable to quantitative nucleic acid analyses such as expression profiling in general comes as a surprise, considering its focus on qualitative nucleic acid detection such as in SNP detection.
In the original technology amplified single stranded DNA is used as a template for primer annealing. For the detection of RNA markers as provided herein, the technology is modified. Either RNA, first strand cDNA or amplified single stranded cDNA might be used as template. If RNA is used as a template, the DNA polymerase is replaced by a (modified) Reverse transcriptase. In an even more preferred embodiment, the invention surprisingly provides the insight that the principle of pyrosequencing, such as for example also known from Ronaghi et al., Analytical Biochemistry 242: 84-89 (1996), can also be applied to the detection and quantitative analyses of two (or even more) templates at once, especially those derived from two or more separate genes instead of only from one gene wherein possibly allelic variation is to be detected.
Sensitivity is even further increased when the detection and quantitative analyses of the template or templates is achieved by employing in step b) one or more additional primers that are designed to hybridise right in front or at least in close proximity of a relative short (i.e 1 to 10, preferably 2 to 5, most preferably 3 nucleotides long) stretch of identical nucleotides on a template to be detected, which stretches are than sequenced during step c). A stretch does not necessarily has to be located immediately adjacent to the selected primer sequence but can be some nucleotides (i.e. 1 or 2) apart. Considering that the quantitative determination than relies on the detection of a plurality (two or more) of said identical stretches per template, which are measured for example by pyrophosphate release during the pyrosequencing reaction, detection sensitivity is further increased. Considering that for example a codon comprises 3 nucleotides, and considering the relative frequency of individual codons in each nucleotide sequence, it is relatively easy to identify such short stretches of identical nucleotides in each template, and design the primer to go along with those stretches to be sequenced. Of course it is not necessary to limit oneself to codons per se in selecting such stretches, and it is preferred to select different stretches per template to be detected. By using additional primers per template in the fashion as described above the invention is not only applicable to expression profiling per se (where two of more templates are (semi)-quantitatively detected), but it also increase the sensitivity of pyrosequencing applied to one template only. Of course, it is not any longer useful in the detection of SNP's, wherein the crux lies in the detection of nucleotide differences in short nucleotide stretches located in close proximity of a primer used.
Clearly, a method as provided herein is also applicable to expression products such as mRNA that derive from different genes. This insight provides exciting possibilities for expression profiling as a whole and is not only limited to the detection of a developmental stage of an organism comprising determining the expression of at least a first gene and a second gene, or fragments thereof, involved in development of said organism. Other applications include the detection or determination of the physiological stage the organism is in, its reactivity towards disease or environmental conditions, and so on.
Other examples are a method according to the invention wherein said analogue comprises deoxyadenosine athiotriphospate (dATPaS). Applying a method according to the invention is particularly useful when said first gene and said second gene are variably expressed, especially during development or after having been subject to environmental stimuli, in this way, changes in time (and thus quality) are easily monitored. As said, a likely option for application of a method according to the application is in the field of plants, however, other organisms are by no means excluded, considering that exactly the same technology serves all needs. The nature and regulation of various processes in such organisms can be determined in detail by combining all available data into a biochemical model. For plants, this is for example useful for determining flower wilting, fruit ripening and leaf senescence. This will undoubtedly lead to a better understanding of the ripening process, it will provide the biomarkers for the diagnostic test and it will provide the tools for marker assisted and molecular breeding towards longer vase life and increased stress resistance. Data generated through above genomics and bio-informatics approach is directly coupled with a practical application. It is expected that this will result in an intensified interest in the use of biotechnology, not only for agricultural and horticultural problems.
A practical benefit will be that an objective high-throughput automated test systems for quality and stage determination in plant products will become available. For ornamentals, but also for other crops or plant products, there are no objective and reliable methods for the determination of internal quality or physiological stage available until now. From a technical point of view, this is very important, as it modifies the use of existing technology for rapid analysis of DNA samples to technology capable of expression analysis. Semi-automated equipment for high-throughput analysis of RNA is not yet available, not even in the medical field, in particularly not where a method according to the invention, allows step b) and/or a) to be performed in one reaction vessel. The use of semi-automated high throughput testing facilities will make it possible to optimize transport chains, to certify batches and to perform tracking and tracing. The technology developed in herein is of use in all sectors of the horticultural or agricultural industry where fresh products are harvested, handled, transported and stored.

Figure legends

Fig 1A. Light emission profile (pyrogram) of a DNA polymerisation reaction carried out in single steps by adding one specific nucleotide at a time. The reaction included one PCR fragment comprising a part of the BGL-gene as a template and one sequencing primer. The order of nucleotide additions is indicated along the y-axis. Arrows indicate the signals which are due to internal folding of the sequencing primer. Peak heights values presented below the figure were used as a quantitative messure for the number of nucleotides reacting at each single step.

Fig 1B As in Fig 1A but now the reaction was performed on a PCR fragment comprising the PRP gene.

Fig 1C As in Fig 1A but now the reaction was performed on a PCR fragment comprising the THA gene.

Fig 1D As in Fig 1A but now the reaction was performed on a PCR fragment comprising the RPL10 gene.

Fig 1E As in Fig 1A but now the reaction was performed on a PCR fragment comprising the RPL12 gene.

Fig 2A. Light emission profile (pyrogram) of a DNA polymerisation reaction carried out in single steps by adding one specific nucleotide at a time. The reaction includes two different PCR fragments, one comprising a part of the THA gene and one comprising a part of the RPL10 gene. One sequence primer annealing to the THA gene fragment and one primer annealing to the RPL10 fragment were added. The order of nucleotide additions is indicated along the y-axis. Peak heights were used as a quantitative measure for the number of nucleotides reacting at each single step. The ratio between the two PCR fragments was calculated from the G 4-mer and C- and T- dimers signals.

Fig 2B. Light emission profile (pyrogram) of a DNA polymerisation reaction carried out in single steps by adding one specific nucleotide at a time. The reaction includes two different PCR fragments, one comprising a part of the PRP gene and one comprising a part of the RPL12 gene. One sequence primer annealing to the PRP gene fragment and one primer annealing to the RPL12 fragment were added. The order of nucleotide additions is indicated along the y-axis. Peak heights were used as a quantitative measure for the number of nucleotides reacting at each single step. The ratio between the two PCR fragments was calculated from the G- and A- dimers signals.

Fig 2C. Light emission profile (pyrogram) of a DNA polymerisation reaction carried out in single steps by adding one specific nucleotide at a time. The reaction includes two different PCR fragments, one comprising a part of the RPL12-gene and one comprising a part of the BGL gene. One sequence primer annealing to the RPL12 gene fragment and one primer annealing to the BGL fragment were added. The order of nucleotide additions is indicated along the y-axis. Peak heights were used as a quantitative measure for the number of nucleotides reacting at each single step. The ratio between the two PCR fragments was calculated from the G- dimers and G- and T- monomers signals.

Detailed description

Examples

Quantification of the expression of different genes involved in the ripeness of cucumber using Pyrosequencing

Primer design

Primers were designed to make it possible to compare a double incorporation (BGL,PRP vs RPL12) or a tetra incorporation (THA vs RPL10) of the same nucleotide (G). The idea was to improve the detectability of a gene with relatively low expression and the two set-ups (2G vs 4G) was done in order to test and enhance the sensitivity. Moreover, the design for BGL,PRP and RPL12 allows for a triplex detection since nucleotide incorporations from each fragment can be separated according to the nucleotide 3' of the primer that precedes double G used for quantification. By placing a primer next to a double G on a fourth fragment it would also be possible to design a tetraplex. Below, the sequencing primers are highlighted and the nucleotides used for quantitative comparison are boxed and underlined.

Variable

>BGL

tatgatcttcctcaagtcttggaagaagagtataaaggcctattgagtgacagagtagtgaaggattttgcagattatgcaga
attttgtttcaaaacgtttggggatagagttaagaattggatgacgtttaacgaaccaagagtcgtggcagctctaggatatga
taatggtttttttgctcctgggaggtgttctaaagcatacgg

Variable

>PRP

atgccccattgacacgctgaagttgggagcgtgtgtggacttgttgggtgggttgatccatatcggaattggtgaccgtacgaa
acaaacttgctgccctgttcttgaaggactagtggatttggatgcggcagtttgtttgtgtaccaccattaaagc

Constitutive

>RPL12

tcaaagagcccgaacgcgaccgcaagaagaccaagaacatcaagcacaatggtaatatctcgcttgacgatgttattgagatt
gctagggttatgcgccccaggtctatggctaaggatctcagtggatccgttaaggagattctcggtacttgcgtttctgttgggtg
tacg

------------------------------------------------------------------------

Variable

>THA

ttacccaaaagatgatgcaaccagcacattcacttgccctggggggaaccaactatagggttgttttctgcccttaaaaccagatt
atatagatataaaaaggaaaccaaacgttacatgaatagttaaagagttgccatatatattatataccttttatataggtatat
atatggtgtaatttgtaataagatttggatatggttggtaaatgagc

Constitutive

>RPL10

cgatgcaaggacagcaacagccagcatgctcaggaggctctccgtcgtgctaagtttaagttccctggtcgtcaaaagatcatt gttagcaggaagtggggattcactaaatttagccgagctgattacctcaagttcaagtcagagaacaagattatgccagatgg tgttaatgctaagct

Materials and Methods

*Template:*

Precipitated RT-PCR products were used. Samples were dissolved in MQ-$H_2O$ according to the strength of the band on the gel, as follows.

| Sample no. | Volume MQ-$H_2O$ (µl) |
|---|---|
| 1.BGL | 90 |
| 2.PRP | 50 |
| 3.THA | 90 |
| 4.RPL10 | 90 |
| 5.RPL12 | 50 |
| 6.RPL10 + THA | 90 |
| 7.RPL12 + PRP | 50 |
| 8.RPL12 + BLC | 90 |

*Sample preparation and Pyrosequencing:*

The sample preparation was carried out according to the Pyrosequencing protocol. Immobilization were done using 20 µl of the dissolved PCR product together with 10µl Dynabeads mixed with 30 µl binding buffer. Annealing was done with 15 pmol sequencing primer. Pyrosequencing and subsequent evaluation was done using the PSQ™ 96 SNP Software v. 1.2 AQ.

The pyrograms for the runs with only one template (BGL, PRP, THA, RPL10 and RPL12) are presented in figure 1. The sequences achieved matches the expected sequences (sequence to analyze) well, except for the BGL (BLC) sequence which is influenced by background generated from extension of the 3'-end of the template, looping back to itself and generating a priming site for the polymerase (see below).

Relative quantification

To be able to determine the relative quantity of each template in the samples with mixed templates, dispensation orders were created that made it possible to achieve isolated peaks from each fragment, see figure 2. For comparison and additional control, other template specific peaks were also compared. These calculations are shown in *italics.*

THA + RPL10

Sequencing primers: TCATTGTTAGCAGGAAG (RPL10) and CATTCACTTGCCCTG (THA)

Sequence to analyze: TGGGGATT (RPL10) and GGGGAACC (THA)

Dispensation order: G(C)TGACT

With this dispensation order, it is possible to distinguish between peaks from RPL10 and THA. The first G-peak corresponds to the 4-mer in the THA-fragment and the second G-peak corresponds to the 4-mer in the RPL10-fragment. *In a similar fashion, the last C-peak correspond to the C-dimer in the THA fragment and the last T-peak correspond to the T-dimer in the RPL10 fragment.*

Calculation of frequencies is done for both the G 4-mer and the C- and T-dimers.

G 4-mer: % RPL10 = (peak height RPL10/(Peak height THA + Peak height RPL10) =
12.3 RLU/(32.6 RLU + 12.3RLU) = 27 %

*C- and T-dimers: % RPL10 = (peak height RPL10/(Peak height THA + Peak height RPL10) =*
*6.9RLU/(14.1 RLU + 6.9RLU) = 33 %*
*% THA = 73 %*
*% RPL10 = 27 %*

PRP + RPL 12

Sequencing primers: AAGAACATCAAGCACAA (RPL12) and GTGGGTTGATCCATAT (PRP)

Sequence to analyze: TGGTAATA (RPL12) and CGGAATTGGT (PRP)

Dispensation order: T(A)GCGATA

With this dispensation order, it is possible to distinguish between peaks from RPL12 and PRP. The first G-peak corresponds to the dimer in the RPL12-fragment and the second G-peak corresponds to the dimer in the PRP-fragment.
*In a similar fashion, the second A-peak corresponds to the A-dimer in the PRP fragment and the last A-peak corresponds to the A-dimer in the RPL12-fragment. Also, the first T-peak corresponds to a monomer from RPL12 and the C-peak to a monomer from PRP.*

Calculation of frequencies is done for both the G- and the A-dimers.

G-dimers: % PRP = (peak height PRP/(Peak height RPL12 + Peak height PRP) =
5.1 RLU/(10.9 RLU + 5.1RLU) = 32 %

*A-dimers: % PRP = (peak height PRP/(Peak height RPL12 + Peak height PRP) =*
*7.0RLU/(11.3RLU + 7.0 RLU) = 38 %*

*T- and C-monomers % PRP = (peak height PRP/(Peak height RPL12 + Peak height PRP) =*
*3.7 RLU/(6.5 RLU + 3.7 RLU) = 36 %*
% PRP = 32 %
% RPL12 = 68 %

RPL 12 + BGL

Sequencing primers: AAGAACATCAAGCACAA (RPL12) and TTGAGTGACAGAGTAGTGA (BGL)

Sequence to analyze: TGGTAATA (RPL12) and AGGATTTTGC (BGL)

Dispensation order: T(C)GAGATAGT

Since there was template background generated from the BGL fragment the G peak intended for quantification has to be omitted since the background will add to the peak height and generate a false result. The intention was to use the first G-peak which corresponds to the G-dimer in the RPL12-fragment and the second G-peak that corresponds to the G-dimer in the BGL-fragment. Instead peaks that should be unaffected by the background have been used for a preliminary quantification. For this, the last G-peak corresponding to the G-monomer in the BGL fragment and the last T-peak corresponding to the T-monomer in the RPL12-fragment were compared, as well as the first T corresponding to a monomer from RPL12 and the second A corresponding to a i monomer from BGL.

Calculation of frequencies is done for both the G-dimers and the G- and T- monomers.

*G-dimers: % BGL = (peak height BGL/(Peak height RPL12 + Peak height BGL) =*
*46.3 RLU/(10.6 RLU + 46.3RLU) = 81 %*

G- and T-monomers: % BGL = (peak height BGL/(Peak height RPL12 + Peak height BGL) =
17.1 RLU/(6.1 RLU + 17.1 RLU) = 74 %

T- and A-monomers: % BGL = (peak height BGL/(Peak height RPL12 + Peak height BGL) =
17.4 RLU/(5.8 RLU + 17.4 RLU) = 75 %
% BGL Average: (75 + 74)/2 = 74 %
% RPL12 = 26 %

The results show that it is possible to generate template specific peaks that can be used for relative comparison of different template levels. Using homopolymeric stretches enhances the sensitivity and makes it possible to determine transcripts expressed at low levels vs those that are well expressed. The results obtained from Pyrosequencing seem to be in agreement with the relative abundance of the templates as deduced from EtBr stained fragments in an agarose gel. However, from the gel picture there seems to be larger differences in expression levels than shown by the Pyrosequencing data. One possible explanation is that fragments of different lengths bind different amounts of EtBr per molecule. Moreover, staining in a gel might not be uniform throughout the gel.

In order to adjust the BGL assay, the forward PCR-primer can be redesigned or just extended in its 5'-end in order to add a couple of mismatching bases.

## Claims

**1.** A method for determining a developmental or physiological stage of an organism comprising determining the expression of at least a first gene and a second gene, or gene fragment, said method comprising

a) providing at least a first nucleic acid template derived from said first gene and a second nucleic acid template derived from said second gene

b) hybridising at least one first primer to said first template and at least one second primer to said second template

c) and determining binding of said primers to said templates in one reaction vessel.

**2.** A method according to claim 1 wherein step c) comprising a sequencing step.

**3.** A method according to claim 2 wherein said sequencing step comprises sequencing amplified DNA.

**4.** A method according to claim 3 wherein said DNA comprises PCR amplified DNA.

**5.** A method according to anyone of claims 1 to 4 wherein a primer comprises a sequencing primer.

**6.** A method according to claim 5 wherein said primer can initiate a sequencing reaction carried out by a DNA-polymerase.

**7.** A method according to claim 6 wherein said DNA polymerase is RNA dependent.

**8.** A method according to claim 6 or 7 wherein a dATP or ddATP analogue is used which is capable of acting as a substrate for said polymerase but incapable of acting as a substrate for a pyrophosphate detection enzyme such as ATP-sulfurylase.

**9.** A method according to claim 8 wherein said analogue comprises deoxyadenosine athiotriphospate (dATPaS)

**10.** A method according to any one of above claims wherein said first gene and said second gene are variably expressed during said development.

**11.** A method according to any one of above claims wherein said organism comprises a plant.

**12.** A method according to any one of above claims wherein step b) is performed in one reaction vessel.

**13.** A method according to any one of above claims wherein step a) is performed in one reaction vessel.

**14.** A method according to any one of above claims wherein step b) further comprises hybridising at least one additional primer to said first template.

**15.** A method according to claim 14 wherein said additional primer is selected to hybridise right in front or at least in close proximity to a short stretch of identical nucleotides on said first template.

**16.** A method according to any one of above claims wherein step b ) further comprises hybridising at least one additional primer to said second template.

**17.** A method according to claim 16 wherein said additional primer is selected to hybridise right in front or at least in close proximity to a relative short stretch of identical nucleotides on said second template.

Figure 1A

BGL

Sequencing primer: TTGAGTGACAGAGTAGTGA

Sequence to analyze: AGGATTTTGC

Dispensation order: (C)A(T)(C)GATGC

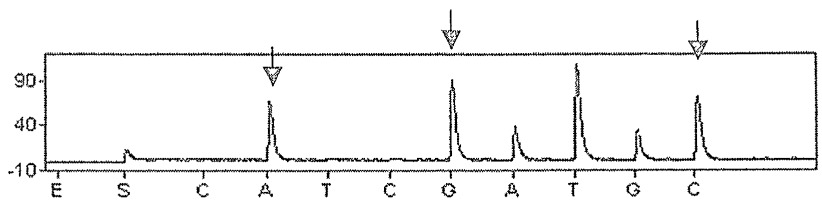


Template loop generating background in the A,G and C peaks denoted by arrows.

AAGCGC ->

ACTTGAGGAAGATCATA -3'

( | || |||||| |||

GAACCTTCTTCTCATATTTCGCGATAACTCACTGTCTCATCACTYCTAAAACGTCTAA

TACGTCTTAAAA... -Biotin-5'

Figure 1B

PRP

Sequencing primer: GTGGGTTGATCCATAT

Sequence to analyze: CGGAATTGGT

Dispensation order: (G)C(T)(A)GATGT

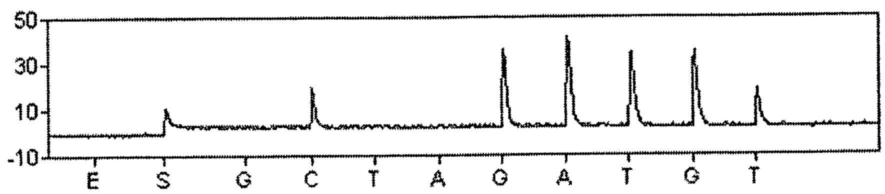

Figure 1C

THA

Sequencing primer: CATTCACTTGCCCTG

Sequence to analyze: GGGGAACCAACT

Dispensation order: (T)(C)G(T)ACACT

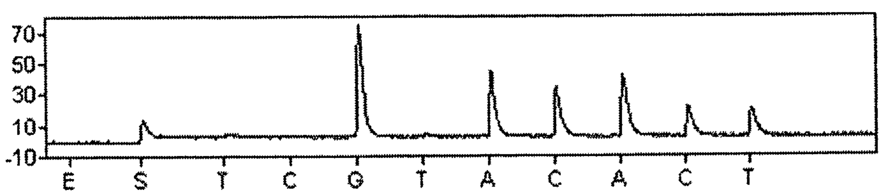

Figure 1D

RPL10

Sequencing primer: TCATTGTTAGCAGGAAG

Sequence to analyze: TGGGGATTCA

Dispensation order: (G)T(C)(A)GATCA

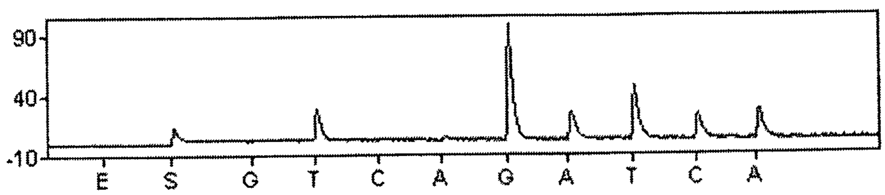

Figure 1E

RPL12

Sequencing primer: AAGAACATCAAGCACAA

Sequence to analyze: TGGTAATA

Dispensation order: (G)T(C)(A)GTATA

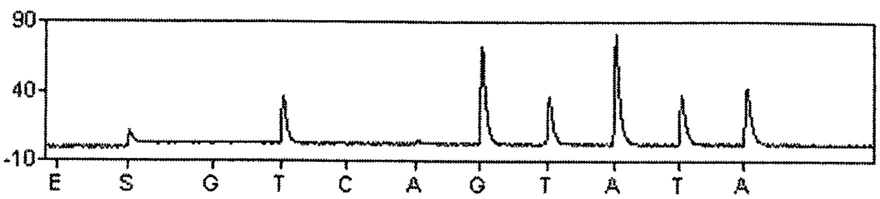

Figure 2A

THA + RPL10

Sequencing primers: TCATTGTTAGCAGGAAG (RPL10) and CATTCACTTGCCCTG (THA)

Sequence to analyze: TGGGGATT (RPL10) and GGGGAACC (THA)

Dispensation order: G(C)TGACT

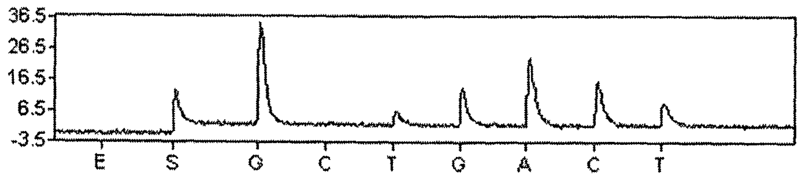


Peak heights from the pyrogram (peak heights in RLU, relative light units):

| S | G | C | T | G | A | C | T |
|---|---|---|---|---|---|---|---|
| 10.6 | 32.6 | 1.1 | 4.7 | 12.3 | 21.8 | 14.1 | 6.9 |

Figure 2B

PRP + RPL 12

Sequencing primers: AAGAACATCAAGCACAA (RPL12) and GTGGGTTGATCCATAT (PRP)

Sequence to analyze: TGGTAATA (RPL12) and CGGAATTGGT (PRP)

Dispensation order: T(A)GCGATA

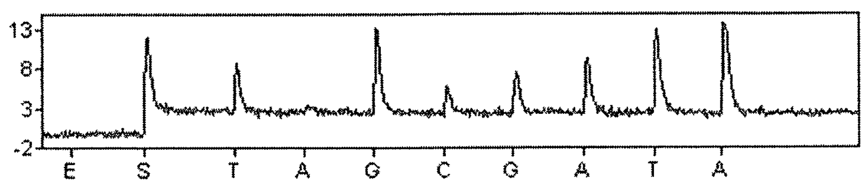


Peak heights from the pyrogram (peak heights in RLU, relative light units):

| S | T | A | G | C | G | A | T | A |
|---|---|---|---|---|---|---|---|---|
| 9.5 | 6.5 | 0.9 | 10.9 | 3.7 | 5.1 | 7.0 | 10.8 | 11.3 |

Figure 2C

RPL 12 + BGL

Sequencing primers: AAGAACATCAAGCACAA (RPL12) and TTGAGTGACAGAGTAGTGA (BGL)

Sequence to analyze: TGGTAATA (RPL12) and AGGATTTTGC (BGL)

Dispensation order: T(C)GAGATAGT

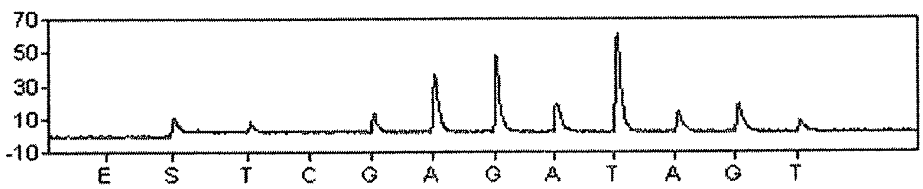


Peak heights from the pyrogram (peak heights in RLU, relative light units):

| S | T | C | G | A | G | A | T | A | G | T |
|---|---|---|---|---|---|---|---|---|---|---|
| 8.5 | 5.8 | 0.6 | 10.6 | 34.2 | 46.3 | 17.4 | 58.1 | 12.0 | 17.1 | 6.1 |

European Patent Office

**PARTIAL EUROPEAN SEARCH REPORT**

which under Rule 45 of the European Patent Convention shall be considered, for the purposes of subsequent proceedings, as the European search report

Application Number EP 01 20 3617

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| X | WO 01 42496 A (DZIEGLEWSKA HANNA ;PYROSEQUENCING AB (SE); LUNDEBERG JOAKIM (SE);) 14 June 2001 (2001-06-14)<br>* whole document *<br>--- | 1-17 | C12Q1/68 |
| X | NYGREN M ET AL: "QUANTIFICATION OF HIV-1 USING MULTIPLE QUANTITATIVE POLYMERASE CHAIN REACTION STANDARDS AND BIOLUMINOMETRIC DETECTION"<br>ANALYTICAL BIOCHEMISTRY, ORLANDO, FL, US, vol. 288, no. 1,<br>1 January 2001 (2001-01-01), pages 28-38, XP000977141<br>ISSN: 0003-2697<br>* whole document *<br>---<br>-/-- | 1-17 | |

**TECHNICAL FIELDS SEARCHED (Int.Cl.7)**

C12Q

**INCOMPLETE SEARCH**

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 22 April 2002 | Aguilera, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# INCOMPLETE SEARCH SHEET C

Application Number
EP 01 20 3617

Claim(s) searched completely:
none

Claim(s) searched incompletely:
1-17

Reason for the limitation of the search:

Claims 1-17 lack clarity and support in the sense of Article 84 EPC. An attempt is made to define the methods by reference to the results to be achieved:
a) being able to determine the expression of at least two genes.
b) being able to determine the binding of primers to templates in one reaction vessel.

The lack of other technical features describing how these effects are to be achieved renders the scope of the claims unclear. This lack of clarity in the present case is such as to render a meaningful search over the whole of the claimed scope impossible. Consequently, the search has been carried out only for those parts of the claims which appear to be clear and supported, namely those parts relating to the methods making use of the pyrosequencing reaction principle to quantify simultaneously two DNA targets in one vessel.

European Patent Office

# PARTIAL EUROPEAN SEARCH REPORT

Application Number
EP 01 20 3617

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | TABARY T ET AL: "HOMOGENEOUS PHASE PYROPHOSPHATE (PPI) MEASUREMENT (H3PIM). \A NON-RADIOACTIVE, QUANTITATIVE DETECTION SYSTEM FOR NUCLEIC ACID SPECIFIC HYBRIDIZATION METHODOLOGIES INCLUDING GENE AMPLIFICATION" JOURNAL OF IMMUNOLOGICAL METHODS, ELSEVIER SCIENCE PUBLISHERS B.V.,AMSTERDAM, NL, vol. 156, no. 1, 1992, pages 55-60, XP000320297 ISSN: 0022-1759 * whole document * --- | 1-17 | |
| Y | KARAMOHAMED SAMER ET AL: "Bioluminometric method for real-time detection of reverse transcriptase activity." BIOTECHNIQUES, vol. 24, no. 2, February 1998 (1998-02), pages 302-306, XP002194466 ISSN: 0736-6205 * whole document * --- | 7 | TECHNICAL FIELDS SEARCHED (Int.Cl.7) |
| Y | WILKINSON JACK Q ET AL: "Identification of mRNAs with enhanced expression in ripening strawberry fruit using polymerase chain reaction differential display." PLANT MOLECULAR BIOLOGY, vol. 27, no. 6, 1995, pages 1097-1108, XP000670213 ISSN: 0167-4412 * page 1098, column 1, paragraph 3 - page 1099, column 1, paragraph 3 * * page 1100, column 1, paragraph 2 - page 1101, column 1, paragraph 1; figure 3 * --- -/-- | 1-17 | |

EPO FORM 1503 03.82 (P04C10)

European Patent Office

# PARTIAL EUROPEAN SEARCH REPORT

Application Number
EP 01 20 3617

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | JONES C S ET AL: "Profiling of changes in gene expression during raspberry (Rubus idaeus) fruit ripening by application of RNA fingerprinting techniques." PLANTA (BERLIN), vol. 211, no. 5, October 2000 (2000-10), pages 708-714, XP002194465 ISSN: 0032-0935 * page 71, column 1, paragraph 1 - column 2, paragraph 1; figure 1 * | 1-17 | |
| A | US 6 210 891 B1 (UHLEN MATHIAS ET AL) 3 April 2001 (2001-04-03) * whole document * | 1-17 | TECHNICAL FIELDS SEARCHED (Int.Cl.7) |
| A | PASTINEN T ET AL: "MINISEQUENCING: A SPECIFIC TOOL FOR DNA ANALYSIS AND DIAGNOSTICS ONOLIGONUCLEOTIDE ARRAYS" GENOME RESEARCH, COLD SPRING HARBOR LABORATORY PRESS, US, vol. 7, no. 6, 1 June 1997 (1997-06-01), pages 606-614, XP000699761 ISSN: 1088-9051 * whole document * | 1-17 | |

EPO FORM 1503 03.82 (P04C10)

## ANNEX TO THE EUROPEAN SEARCH REPORT ON EUROPEAN PATENT APPLICATION NO. EP 01 20 3617

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2002

| Patent document cited in search report | | Publication date | Patent family member(s) | | | Publication date |
|---|---|---|---|---|---|---|
| WO 0142496 | A | 14-06-2001 | AU | 2192801 | A | 18-06-2001 |
| | | | WO | 0142496 | A2 | 14-06-2001 |
| US 6210891 | B1 | 03-04-2001 | AU | 4391897 | A | 17-04-1998 |
| | | | DE | 69706430 | D1 | 04-10-2001 |
| | | | EP | 0932700 | A1 | 04-08-1999 |
| | | | WO | 9813523 | A1 | 02-04-1998 |
| | | | JP | 2001501092 | T | 30-01-2001 |

EPO FORM P0459

For more details about this annex : see Official Journal of the European Patent Office, No. 12/82

SEQUENCE LISTING

<110> GT Diagnostics B.V.

<120> Expression profiling

<130> P56791EP00

<140> 01203617.4
<141> 2001-09-25

<160> 20

<170> PatentIn Ver. 2.1

<210> 1
<211> 209
<212> DNA
<213> cucumis

<220>
<221> misc_feature
<222> (1)..(209)
<223> /note="Template BGL"

<400> 1
tatgatcttc ctcaagtctt ggaagaagag tataaaggcc tattgagtga cagagtagtg 60
aaggattttg cagattatgc agaattttgt ttcaaaacgt ttggggatag agttaagaat 120
tggatgacgt ttaacgaacc aagagtcgtg gcagctctag gatatgataa tggttttttt 180
gctcctggga ggtgttctaa agcatacgg 209

<210> 2
<211> 159
<212> DNA
<213> cucumis

<220>
<221> misc_feature
<222> (1)..(159)
<223> /note="Template PRP"

<400> 2
atgccccatt gacacgctga agttgggagc gtgtgtggac ttgttgggtg ggttgatcca 60
tatcggaatt ggtgaccgta cgaaacaaac ttgctgccct gttcttgaag gactagtgga 120
tttggatgcg gcagtttgtt tgtgtaccac cattaaagc 159

<210> 3
<211> 173
<212> DNA
<213> cucumis

<220>
<221> misc_feature
<222> (1)..(173)
<223> /note="Template RPL 12"

<400> 3
tcaaagagcc cgaacgcgac cgcaagaaga ccaagaacat caagcacaat ggtaatatct 60
cgcttgacga tgttattgag attgctaggg ttatgcgccc caggtctatg gctaaggatc 120
tcagtggatc cgttaaggag attctcggta cttgcgtttc tgttgggtgt acg 173

<210> 4
<211> 216
<212> DNA
<213> cucumis

```
<220>
<221> misc_feature
<222> (1)..(216)
<223> /note="Template THA"

<400> 4
ttacccaaaa gatgatgcaa ccagcacatt cacttgccct gggggaacca actatagggt 60
tgttttctgc ccttaaaacc agattatata gatataaaaa ggaaaccaaa cgttacatga 120
atagttaaag agttgccata tatattatat accttttata taggtatata tatggtgtaa 180
tttgtaataa gatttggata tggttggtaa atgagc                           216


<210> 5
<211> 182
<212> DNA
<213> cucumis

<220>
<221> misc_feature
<222> (1)..(182)
<223> /note="Template RPL10"

<400> 5
cgatgcaagg acagcaacag ccagcatgct caggaggctc tccgtcgtgc taagtttaag 60
ttccctggtc gtcaaaagat cattgttagc aggaagtggg gattcactaa atttagccga 120
gctgattacc tcaagttcaa gtcagagaac aagattatgc cagatggtgt taatgctaag 180
ct                                                                182


<210> 6
<211> 17
<212> DNA
<213> Artificial Sequence

<220>
<223> Description of Artificial Sequence: primer RPL10

<220>
<221> misc_feature
<222> (1)..(17)

<400> 6
tcattgttag caggaag                                                 17


<210> 7
<211> 15
<212> DNA
<213> Artificial Sequence

<220>
<223> Description of Artificial Sequence: primer THA

<220>
<221> misc_feature
<222> (1)..(15)

<400> 7
cattcacttg ccctg                                                   15


<210> 8
<211> 8
<212> DNA
<213> cucumis

<220>
```

<221> misc_feature
<222> (1)..(8)
<223> /note="Part of template RPL10 = sequence to analyze"

<400> 8
tggggatt 8

<210> 9
<211> 8
<212> DNA
<213> cucumis

<220>
<221> misc_feature
<222> (1)..(8)
<223> /note="Part of template THA = sequence to analyze"

<400> 9
ggggaacc 8

<210> 10
<211> 17
<212> DNA
<213> Artificial Sequence

<220>
<223> Description of Artificial Sequence: primer RPL12

<220>
<221> misc_feature
<222> (1)..(17)

<400> 10
aagaacatca agcacaa 17

<210> 11
<211> 16
<212> DNA
<213> Artificial Sequence

<220>
<223> Description of Artificial Sequence: primer PRP

<220>
<221> misc_feature
<222> (1)..(16)

<400> 11
gtgggttgat ccatat 16

<210> 12
<211> 8
<212> DNA
<213> cucumis

<220>
<221> misc_feature
<222> (1)..(8)
<223> /note="Part of template RPL12 = sequence to analyze"

<400> 12
tggtaata 8

<210> 13
<211> 10
<212> DNA
<213> cucumis

<220>
<221> misc_feature
<222> (1)..(10)
<223> /note="Part of template PRP = sequence to analyze"

<400> 13
cggaattggt 10

<210> 14
<211> 19
<212> DNA
<213> Artificial Sequence

<220>
<223> Description of Artificial Sequence: primer BGL

<220>
<221> misc_feature
<222> (1)..(19)

<400> 14
ttgagtgaca gagtagtga 19

<210> 15
<211> 10
<212> DNA
<213> cucumis

<220>
<221> misc_feature
<222> (1)..(10)
<223> /note="Part of template BGL = sequence to analyze"

<400> 15
aggattttgc 10

<210> 16
<211> 6
<212> DNA
<213> cucumis

<220>
<221> misc_feature
<222> (1)..(6)
<223> /note="Part of template BGL, generating background"

<400> 16
aaggcc 6

<210> 17
<211> 17
<212> DNA
<213> cucumis

<220>
<221> misc_feature

```
<222> (1)..(17)
<223> /note="Part of template BGL, generating
      background"

<400> 17
acttgaggaa gatcata                                                    17


<210> 18
<211> 70
<212> DNA
<213> Artificial Sequence

<220>
<223> Description of Artificial Sequence: extension of
      the 3' end of template BGL

<220>
<221> misc_feature
<222> (1)..(70)

<400> 18
gaaccttctt ctcatatttc cggataactc actgtctcat cactyctaaa acgtctaata 60
cgtcttaaaa                                                            70


<210> 19
<211> 12
<212> DNA
<213> cucumis

<220>
<221> misc_feature
<222> (1)..(12)
<223> /note="Part of template THA = sequence to analyze"

<400> 19
ggggaaccaa ct                                                         12


<210> 20
<211> 10
<212> DNA
<213> cucumis

<220>
<221> misc_feature
<222> (1)..(10)
<223> /note="Part of template RPL10 = sequence to
      analyze"

<400> 20
tggggattca                                                            10
```